Europäisches Patentamt

European Patent Office

Office européen des brevets

(19)

(11) Veröffentlichungsnummer: **0 106 179**
**B1**

(12) **EUROPÄISCHE PATENTSCHRIFT**

(45) Veröffentlichungstag der Patentschrift :
28.05.86

(21) Anmeldenummer : 83109202.8

(22) Anmeldetag : 16.09.83

(51) Int. Cl.⁴ : **C 07 K 15/06, C 12 N 5/00,**
**C 12 P 21/00, A 61 K 35/12,**
**A 61 K 37/02**

(54) Homogenes Human-Interleukin-2 und Verfahren zu seiner Herstellung.

(30) Priorität : 16.09.82 US 418927

(43) Veröffentlichungstag der Anmeldung :
25.04.84 Patentblatt 84/17

(45) Bekanntmachung des Hinweises auf die Patenterteilung : 28.05.86 Patentblatt 86/22

(84) Benannte Vertragsstaaten :
AT BE CH DE FR GB IT LI LU NL SE

(56) Entgegenhaltungen :
NATURE, Band 302, 24. März 1983, London T. TANIGUCHI et al. "Structure and expression of acloned cDNA for human interleukin-2", Seiten 305-310
Chemical Abstracts Band 97, Nr. 17, 25. Oktober 1982, Columbus, Ohio, USA K. WELTE et al. "Purification of human interleukin 2 to apparent homogeneity and its molecular heterogeneity", Seite 516, Spalte 1, Abstract Nr. 142913b
Chemical Abstracts Band 98, Nr. 9, 28. Februar 1983, Columbus, Ohio, USA J. GERARD et al. "Chromatofocusing as a tool for the characterization and partial purification of human interleukin-2", Seite 462, Spalte 1, Abstract Nr. 70071w
Chemical Abstracts Band 93, Nr. 24, 15. Dezember 1980, Columbus, Ohio, USA T.F. KUSNETSOVA et al. "Effect of anion-active surfactants and normal fatty acids on the structural formation of globular adsorbents", Seite 364, Spalte 2, Abstract Nr. 226258x

(73) Patentinhaber : F. HOFFMANN-LA ROCHE & CO.
Aktiengesellschaft
CH-4002 Basel (CH)

(72) Erfinder : Stern, Alvin S.
110 Kensington Terrace
Passaic Park, N.J. (US)

(74) Vertreter : Lederer, Franz, Dr. et al
Patentanwälte Dr. Lederer Franz Meyer-Roxlau Reiner F. Lucile-Grahn-Strasse 22
D-8000 München 80 (DE)

**Beschreibung**

Interleukin-2 (IL-2) ist ein lösliches Protein, das in der Lage ist, die Lymphozyten-Reaktivität zu modulieren und das bisher durch Stimulierung von Mäuse-, Ratten- oder Human-Lymphozytenzellen mit einem Mitogen, wie Phytohämagglutinin (PHA) und Concanavalin A (Con A) hergestellt wurde [vgl. z. B. Gillis et al. : Nature *268*, 154 (1977) ; J. Immunol. *120*, 2027 (1978) ; ibid. *124*, 1954 (1980) ; ibid. *125*, 2570 (1980)].

Gereinigtes IL-2 aus normalen T-Lymphozyten der Maus, Ratte oder des Menschen, besitzt nachgewiesenermassen die folgenden unterschiedlichen biologischen Aktivitäten : (1) ausgeprägte Verstärkung der Thymozyten-Mitogenese ; (2) Förderung der Langzeit-Proliferation Antigen-spezifischer T-Helfer- oder T-Killer-Zell-Linien in vitro und (3) Induktion von cytotoxischer T-Lymphozyten-Reaktivität und von Antikörperbildung-auslösenden Zell-Antworten in Milzzellkulturen von nackten Mäusen. Diese nachgewiesenen biologischen Aktivitäten zeigen, dass IL-2 Immunantworten verstärken und die zellgebundene und humorale Immunität immun-geschwächter T-Zellpopulationen (Milzzellen nackter Mäuse) auf ein normales Niveau anheben kann. Darüberhinaus lassen diese Ergebnisse vermuten, dass die IL-2-Produktion und -Aktivität wichtige Parameter für immunologische Funktionen sind, die zur klinischen Diagnose von Abweichungen im Immunsystem herangezogen werden können. Schliesslich verdeutlicht die Tatsache, dass Human-IL-2 (HIL-2) eine in-vitro-Proliferation Antigen-spezifischer T-Killerzellen von Mensch, Maus und Ratte bewirkt, seine Bedeutung als Forschungsreagens.

Obgleich die Gewinnung von HIL-2 aus Lectin-stimulierten Human-Lymphozyten der Milz und des peripheren Bluts in bestimmten Medien von verschiedenen Autoren beschrieben wurde, handelt es sich stets nur um niedrige IL-2-Konzentrationen, bei aufwendiger Reinigung durch Fraktionierung grosser Volumina. Tatsächlich stand HIL-2 bisher nicht in genügender Menge zur Verfügung für in-vivo-Experimente oder die Aufklärung seiner molekularen Struktur.

Von Gillis und Watson [J. Exp. Med. *152*, 1709-1719 (1980)] wurde die Produktion von HIL-2 durch Induktion einer malignen neoplastischen Zellinie (Jurkat-FHCRC) mit einem T-Zellmitogen wie PHA, gegebenenfalls in Gegenwart eines Phorbolesters wie Phorbolmyristinacetat (PMA), beschrieben. Teilweise Reinigung des IL-2 wurde durch ein Verfahren erreicht, das Ammoniumsulfat-Fällung/Dialyse, Gelfiltration (Sephadex® G-100), Ionenaustauschchchromatographie (DEAE-Cellulose), isoelektrische Fo-kussierung und analytische Natriumdodecylsufat-Polyacrylamid-Gelelektrophorese (SDS-PAGE) umfasste. Der Hauptanteil der biologischen IL-2-Aktivität wurde elektrophoretisch aus einer Proteinbande isoliert (eine von 9 bis 16 aufgetrennten Banden) die ein Molekulargewicht von etwa 14,000 Dalton aufwies. Vgl. auch Frank et al., J. Immunol. *127*, 2361 (1981) und Watson et al., Lymphokines *6*, 95 (1982).

Mier und Gallo [J. Immunol. *128*, 1122 (1982) ; Lymphokines *6*, 137 (1982)] berichteten über die Reinigung von IL-2 aus normalen Lymphozyten, wobei ein « nahezu homogenes Material » erhalten wurde, unter Verwendung von SDS-Gelelektrophorese als letztem Schritt nach einer Folge von Anionenaustauschchromatographie- und Gelfiltrations-Schritten. Ihr Produkt hatte ein Molekulargewicht von 13,000 gemäss SDS-PAGE und 20-25,000 gemäss Gelfiltration und einen isoelektrischen Punkt von 6,8. Dieses Material war selbst bei —70 °C sehr instabil und erforderte, zwecks Stabilisierung der Aktivität, den Zusatz von Rinderserumalbumin (BSA).

Stadler und Oppenheim [Lymphokines *6*, 117 (1982)] beschreiben gereinigtes IL-2 aus einkernigen Zellen peripheren Bluts, der Mandeln und der Milz, die sich nach der Reinigung durch Säulenchromatographie, Gelfiltration und Elektrofokussierung durch unterschiedliche Ladung voneinander unterscheiden. Es wurden drei Spezies gefunden mit pIs von 6,5, 7,2 und 8,2. Die unterschiedliche Ladung kann ihren Grund in unterschiedlicher Glycosylierung haben.

Schliesslich haben Robb und Smith [Mol. Immunol. *18*, 1987-94 (1981)] TCGF (= IL-2) aus der menschlichen T-Leukämie-Zellinie JURKAT isoliert, der einheitlich hinsichtlich der Ladung und der Grösse ist. Das gleiche Resultat wurde von Robb et al. in J. Exp. Med. *184*, 1455-74 (1981) berichtet.

Die vorliegende Erfindung betrifft HIL-2, das aus induzierten malignen menschlichen Zellen stammt und bis zur Homogenität gereinigt wurde, unter Anwendung verschiedener HPLC-Schritte an Umkehrphasen. Das Verfahren ist dadurch gekennzeichnet, dass man

a) eine rohe Lösung von HIL-2 an einer Umkehrphasen-HPLC (High Performance Liquid Chromatography)-Saüle auf der Basis von Kieselgel, das kovalent gebundene Methyl- oder Octylgruppen enthält, unter HPLC-Bedingungen chromatographiert, wobei die HIL-2-Aktivität mit einem n-Propanol-Gradienten von 0 bis 60 % (v/v) in einem Puffer eluiert und zu einer Fraktion vereinigt wird, und

b) diese Fraktion an einer weiteren Umkehrphasen-HPLC-Säule auf der Basis von Kieselgel, das kovalent gebundene Diphenylgruppen enthält, unter HPLC-Bedingungen chromatographiert, wobei die HIL-2-Aktivität mit einem n-Propanol-Gradienten von 20 bis 60 % (v/v) in einem Puffer eluiert und zu einer Fraktion vereinigt wird.

In denjenigen Fällen, in denen das verwendete Ausgangsmaterial einen sehr niedrigen HIL-2-Titer aufweist, kann es notwendig werden, den ersten und/oder zweiten HPLC-Schritt zu wiederholen, um eine Reinigung bis zur Homogenität zu erreichen. Das homogene HIL-2 ist gekennzeichnet durch eine spezifische Aktivität von etwa $1,4 \times 10^9$ E/mg ein pI von 5,68 und die folgende Aminosäurezu-sammensetzung :

| | | | | | |
|---|---|---|---|---|---|
| Asp | 12 | Ala | 7 | Tyr | 3 |
| Thr | 10 | Cys | 4-5 | Phe | 5 |
| Ser | 8 | Val | 6 | His | 4 |
| Glu | 16-17 | Met | 4 | Lys | 8 |
| Pro | 8-9 | Ile | 7 | Arg | 4 |
| Gly | 8 | Leu | 16 | | |

Rohes HIL-2 kann dadurch erhalten werden, dass man maligne menschliche neoplastische Zellen, beispielsweise menschliche Leukämie- und Lymphom-Zellen, in vitro in einem Serum kultiviert, das verschiedene Additive enthält. Die Kultur wird durch ein T-Zellmitogen, beispielsweise PHA, stimuliert, wodurch ein Ueberstand erhalten wird, der IL-2 enthält. Eine bestimmte Zeit nach der Inkubation, z. B. nach 24 Stunden, wird der Ueberstand gesammelt und aufgearbeitet zwecks Konzentrierung von IL-2. Als Induktionsmittel kann man auch einen Phorbolester, z. B. PMA, allein oder in Kombination mit einem T-Zellmitogen, wie PHA oder Con A, zwecks Erhöhung der IL-2-Produktion verwenden. Weitere Einzelheiten siehe Gillis et al., J. Exp. Med. *152,* 1709 (1980).

Als Zellinien, die zur Gewinnung von rohem HIL-2 verwendet werden können, kommen verschiedene T- und B-Zellinien sowie verschiedene T-Lymphom-Zellinien in Frage. Die Zellinien wurden entweder durch spontanes Auftreten erhalten, durch Virusinfektion oder mittels chemischer Carzinogene. Eine für die vorliegende Erfindung bevorzugte Zellinie ist die menschliche leukämische T-Zellinie, die von Gillis et al. als Jurkat-FHCRC bezeichnet wird, insbesondere ein Subklon dieser Linie, der H33HJ-JAI bezeichnet wird. Das Verfahren der vorliegenden Erfindung kann ebenfalls zur Reinigung von IL-2 aus peripheren Blutlymphozyten verwendet werden.

Als Kulturmedien für die Gewinnung von rohem HIL-2 aus den vorstehend genannten Zellinien können im Handel erhältliche Medien verwendet werden, beispielsweise das von Dulbecco modifizierte Eagle-Medium, RPMI-Medium oder Click-Medium. Als Additive, die dem Kulturmedium allein oder in Kombination zugesetzt werden können, kommen Penicillin, Streptomycin, Gentamycin, frisches L-Glutamin, HEPES-Puffer, $NaHCO_3$, fötales Kälberserum (FCS) oder normales menschliches Serum in Betracht. Die Anfangszelldichte, insbesondere bei Verwendung der Jurkat-FHCRC-Zellen, sollte in der Grössenordnung von etwa $5 \times 10^5$ bis $1 \times 10^7$ Zellen/ml, vorzugsweise bei etwa $1 \times 10^6$ Zellen/ml liegen.

Ferner sollten etwa die folgenden Bedingungen eingehalten werden : Eine Temperatur von etwa 35-38 °C, ein pH von 7,0 bis 7,4 und feuchte Luft mit einem Gehalt von etwa 5-10 % $CO_2$. Die PHA-Mitogen-Konzentration sollte in der Grössenordnung von 0,5-2,0 Volumenprozent, vorzugsweise 1 Volumenprozent, liegen.

Die Menge des durch Stimulierung maligner menschlicher Zellen mit einem Pflanzen-Mitogen erhaltenen HIL-2 ist zeitabhängig. Maximale Mengen werden etwa 16-24 Stunden nach Stimulierung mit PHA erreicht. Ein IL-2-Assay, mit dem die IL-2-Produktion in der Zellkultur oder das Reinigungsverfahren verfolgt werden kann, basiert auf der Fähigkeit der Probe T-Zell-Proliferation zu induzieren. Die Bestimmung erfolgt durch Messung der Einbaus von tritiiertem Thymidin. Die Einheit der Aktivität ist definiert als die Anzahl µl einer T-Zellkultur, die 50 % des maximalen Thymidineinbaus induziert. Weitere Einzelheiten über den Assay siehe bei Gillis et al., J. Immunol. *120,* 2027 (1978).

Die Herstellung eines geeigneten Ausgangsmaterials für die erfindungsgemässe Reinigung mittels HPLC umfasst die Fällung von IL-2 aus den durch Zentrifugieren erhaltenen Zellkulturüberständen mittels Ammoniumsulfat durch Sättigung bis zu 85 %. Der Zusatz von Ammoniumsulfat zum Ueberstand erfolgt in trockener Form unter leichtem Rühren über eine längere Zeit, beispielsweise während 12 Stunden. Nach Erreichung einer 85 %igen Sättigung wird noch eine Weile in der Kälte gerührt, dann wird der Proteinniederschlag durch Zentrifugieren abgetrennt und in sterilem doppelt destilliertem Wasser resuspendiert.

Gemäss einer Verfahrensvariante wird das rohe resuspendierte IL-2 der Ionenaustauschchromatographie unterzogen. Eine geeignete Säule für diesen Zweck ist CM Biogel® A (LKB-produkter, Bromma, Schweden). Vorzugsweise wird die Säule mit fötalem Kälberserum vorbehandelt, um nicht-spezifische Bindungsstellen auf dem Harz vor Aufgabe der IL-2-enthaltenden Probe zu blockieren. Die Elution von der Säule erfolgt mittels gepuffertem Salzgradienten. Ein geeigneter Gradient ist 50 mM-0,5 M NaCl in HEPES, pH 5,5. Schliesslich wird mit 0,5 M NaCl-HEPES, pH 5,5, gewaschen, um sicherzustellen, dass die gesamte IL-2-Aktivität eluiert wurde.

Die vereinigten aktiven Fraktionen der Ionenaustauschchromatographie stellen ein bevorzugtes Ausgangsmaterial für die folgende erfindungsgemässe Reinigung durch HPLC dar. Erhält man jedoch nach der Ammoniumsulfatfällung Ueberstände mit einem sehr hohen IL-2-Titer, so kann man dieses Material direkt der HPLC unterwerfen. in diesem Falle kann es jedoch nötig werden, dass man einen oder beide HPLC-Schritte wiederholt, um eine Reinigung des IL-2 bis zur Homogenität zu erreichen.

Bei der erfindungsgemässen HPLC bedient man sich einer Umkehrphase auf der Basis von Kieselgel, das kovalent gebundene Methyl-, Octyl- oder Diphenylgruppen enthält, mit einer Porengrösse von mindestens etwa 150 Å. Geeignete Umkehrphasen-HPLC-Säulen, die erfindungsgemäss verwendet werden können, sind im Handel erhältlich. Besonders geeignet sind Protesil-Säulen der Firma Whatman Separations Inc., Clifton, N. J., USA.

So ist Whatman Protesil 300 Methyl ein Material, das über Si-O-Si-Bindungen an die Oberfläche von Kieselgel mit einem Porendurchmesser von 30 nm (300 Å) gebundene Trimethylsilylgruppen enthält und das eine mittlere Teilchengrösse von 8 nm aufweist. Whatman Protestil Magnum 9 Octyl ist Kieselgel mit einem Porendurchmesser von 15 nm (150 Å), das kovalent gebundene Octylgruppen enthält, während Whatman Protesil Diphenyl Kieselgel mit einem Porendurchmesser von 30 nm (300 Å) und kovalent gebundenen Diphenylgruppen darstellt.

Die Elution der Proteine von den HPLC-Säulen kann in an sich bekannter Weise erfolgen : Ein geeignetes Elutionsprozedere zur Entfernung der an die Methyl- oder Octyl-Säule gebundenen Proteine im ersten HPLC-Verfahrensschritt der vorliegenden Erfindung besteht in der Anwendung eines ansteigenden Konzentrationsgradienten des n-Propanols ; Der Gradient geht von 0 bis 60 % (V/V) in einem Puffer vorzugsweise Pyridin-Essigsäure, pH 4,0.

Aehnliche Elutionsbedingungen werden im zweiten HPLC-Schritt für die Diphenyl-Säule verwendet, wobei jedoch ein n-Propanol-Gradient von 20-60 % (V/V) benutzt wird. Die Elution des Proteins wird zweckmässigerweise mit einem der üblichen Detektor-Systeme überwacht, beispielsweise mit dem von Kenny et al. beschriebenen System (Methods Enzymol. *78*, 435, (1981)).

Durch die erfindungsgemässe HPLC, entweder in zwei Schritten oder, sofern das Ausgangsmaterial nur einen niederen Titer besitzt, nach ein- oder mehrmaliger Wiederholung des ersten und/oder zweiten Schritts, wird HIL-2 mit guter Ausbeute in homogener Form erhalten, in einem einzigen symmetrischen Peak der Bioaktivität.

Die Herstellung von homogenem HIL-2 ermöglicht zum ersten Mal die Bestimmung der Aminosäurezusammensetzung sowie die Bestimmung der Aminosäurezusammensetzung sowie die Bestimmung der Aminosäuresequenz, was wiederum eine wichtige Hilfe für die Klonierung des HIL-2-Gens und die Herstellung grosser Mengen von reinem rekombinantem HIL-2 für ausgedehnte klinische Versuche und weite medizinische Anwendung dieser Substanz bedeutet. Die Verfügbarkeit von homogenem HIL-2 wird genaue biologische Untersuchungen seiner Aktivität ermöglichen, frei von Verunreinigungen durch verschiedene andere Lymphokine, die bekanntermassen gleichzeitig mit IL-2 während der Induktionsphase entstehen. Obgleich in der Literatur behauptet wurde, dass hochreines oder nahezu homogenes IL-2 bereits erhalten wurde, stellte sich bei der Untersuchung von derartigem, als eine einzige Bande bei SDS-PAGE anfallendem Material mit dem erfindungsgemässen Verfahren heraus, dass diese Bande in eine Vielzahl von Proteinpeaks aufgelöst werden konnte, die keine IL-2-Aktivität besitzen. Aufgrund dieser Untersuchungen ist davon auszugehen, dass derartige Präparate eine Reinheit von höchstens 10 %, in einigen Fällen sogar von weniger als 1 % aufweisen.

Tests mit teilweise gereinigten IL-2-Präparaten, die von Jurkat-FHCRC-Zellmaterial gewonnen wurden, zeigten, dass dieses nur teilweise gereinigte Lymphokin keine Induktion der Proliferation von frischen peripheren Blutlymphozyten bewirkt, dies allerdings bei T-Lymphozyten-Populationen tut, die vorher mit Lectinen oder spezifischen Antigenen stimuliert wurden. Darüberhinaus wurde festgestellt, dass teilweise gereinigtes HIL-2 die Proliferation von Antigen-spezifischen T-Effektorzellen nicht nur des Menschen sondern auch der Maus fördert. Da aus Jurkat-FHCRC-Zellen isoliertes, nur teilweise gereinigtes HIL-2 bereits als nützliches medizinisches Mittel beschrieben und im Handel erhältlich ist, bietet die ähnliche Verwendung des erfindungsgemässen homogenen HIL-2 dem Fachmann keinerlei Schwierigkeiten.

Die Erfindung wird durch das folgende Beispiel illustriert :

Beispiel

IL-2-Produktion

H33HJ-JAl-Zellen (ATCC Nr. CRL-8163, hinterlegt am 26. August 1982), ein Klon der Jurkat-FHCRC-Zellinie, wurden in RPMI 1640-Medium, das mit 10 % fötalem Kälberserum, 50 E/ml Penicillin, 50 µg/ml Streptomycin, 50 µg/ml Gentamycin und 300 µg/ml frischem L-Glutamin ergänzt war, bei 37 °C in feuchter Luft-Atmosphäre, 5 % $CO_2$ enthaltend, gezüchtet. Bei einer Zelldichte von 8-10 × $10^5$ Zellen/ml Medium wurden die Zellen abzentrifugiert und in frischem RPMI 1640-Medium ohne Serum aber 50 E/ml Penicillin, 50 µg/ml Streptomycin, 50 µg/ml Gentamycin und 300 µg/ml frisches L-Glutamin enthaltend, auf eine Konzentration von 2 × $10^6$ Zellen/ml resuspendiert. Stimulation erfolgte durch Zusatz von 1 Volumenprozent PHA und 10 ng/ml Phorbolmyristinacetat (PMA). Nach 24-stündiger Inkubation bei 37 °C in einer feuchten Luft mit 5 % $CO_2$ wurden die induzierten Zellen zentrifugiert und die Ueberstände als rohes Ausgangsmaterial verwendet.

Die vereinigten Ueberstände aus drei Zellkulturansätzen (Nr. 1-3) mit einem Volumen von 900 ml, 900 ml und 1050 ml bzw. einem Gesamtvolumen von 2850 ml und einer Gesamtaktivität von 7,125 × $10^6$ Einheiten (bestimmt nach Gillis et al., J. Immunol. *120*, 2027 (1978)), wurden innerhalb von 12 Stunden sukzessive unter leichtem Rühren mit trockenem Ammoniumsulfat bis zu einer Sättigung von 85 % versetzt. Es wurde noch 24 Stunden bei 4 °C weitergerührt und dann 30 Minuten mit 10000 × G zentrifugiert. Der Ueberstand wurde abgetrennt und der Rückstand in 40 ml doppelt destilliertem sterilem Wasser resuspendiert. Die erhaltene Lösung enthielt 205000 E/ml IL-2 bzw. insgesamt 8,2 × $10^6$ Einheiten.

In analoger Weise wurden weitere Ansätze (Nr. 4-7) bearbeitet. Die Ergebnisse sind in der folgenden Tabelle I zusammengefasst.

4

Tabelle I

| Ansatz Nr. | Ueberstand Volumen [ml] | Gesamte Aktivität [E/ml] | Total Einh. $\times 10^6$ | Resuspension Volumen [ml] | Aktivität [E/ml] | Total Aktivität [U $\times 10^6$] |
|---|---|---|---|---|---|---|
| 4 | 1200 | | | | | |
| 5 | 250 | 6500 | 9,425 | 55 | 200000 | 11 |
| 6 | 1200 | | | | | |
| 7 | 700 | 6500 | 12,35 | 80 | 163840 | 13,107 |

0 106 179

Ionenaustauschchromatographie an CM Biogel A

Eine 100 ml CM Biogel A-Säule wurde mit 0,05 M NaCl-HEPES, pH 5,5, äquilibriert und anschliessend mit 100 ml 0,005 M NaCl-HEPES, pH 5,5, enthaltend 10 % FCS, behandelt.Diese Behandlung diente dem Zweck, nicht-spezifische Bindungsstellen, die HIL-2 binden könnten, zu blockieren. An die Säule gebundene Serumproteine wurden mit 500 ml 0,5 M NaCl-HEPES, pH 5,5, eluiert. Schliesslich wurde die Säule nochmals mit 1000 ml 0,05 M NaCl äquilibriert.

Die Säule wurde mit den vereinigten Suspensionen der Ammoniumsulfatfällung (Ansätze 1-5) beschickt und anschliessend mit 200 ml 0,05 M NaCl-HEPES, pH 5,5, gewaschen. Anschliessend wurde mit 500 ml NaCl-HEPES, pH 5,5, mit einem Salzgradienten von 50 mM bis 0,5 M eluiert und schliesslich mit 200 ml 0,5 M NaCl-HEPES, pH 5,5, gewaschen, um sicherzustellen, dass das gesamte gebundene IL-2 von der Säule eluiert war. Es wurden Fraktionen zu jeweils 5 ml gesammelt und jede dritte Fraktion wurde auf IL-2-Aktivität untersucht. Die aktiven Fraktionen wurden vereinigt und man erhielt insgesamt 36 ml mit einer Aktivität von 983040 E/ml bzw. eine Totalaktivität von $35,39 \times 10^6$ Einheiten.

In analoger Weise wurde aus den Ansätzen 6 und 7 215 ml eines Eluats mit einer Aktivität von 80000 E/ml bzw. einer Gesamtaktivität von $17,2 \times 10^6$ Einheiten erhalten. 130 ml hiervon wurden mit den 36 ml der ersten Fraktion zu insgesamt 166 ml roher IL-2-Lösung vereinigt, mit einer gemessenen Gesamtaktivität von $10,4 \times 10^6$ Einheiten.

Umkehrphasen-HPLC an der Octyl-Säule

Die gemäss vorstehender Beschreibung erhaltenen 166 ml wurden direkt auf eine 9,4 × 250 ml Protesil Magnum 9 Octyl-Säule (Whatman Separations Inc., Clifton, N. J., USA) gegeben. Die Säule wurde mit 50 ml 0,9 M Essigsäure/0,2 M Pyridin, pH 4,0, gewaschen. Die Elution des Proteins erfolgte mit einem n-Propanol-Gradienten von 0-60 % (V/V) in 0,9 M Essigsäure/0,2 M Pyridin (pH 4), während 8 Stunden. Mit einem automatischen Fluoreszenzdetektor unter Verwendung von Fluorescamin wurde das Protein im Eluat gemessen. Die Ausbeute an biologischer Aktivität lag bei $7,13 \times 10^6$ Einheiten (etwa 70 %).

Umkehrphasen-HPLC an der Diphenyl-Säule

Die Fraktionen von der Octyl-Säule mit den Hauptaktivitäts-Peaks wurden 1 : 1 (V/V) mit 0,9 m Essigsäure/0,2 M Pyridin, pH 4,0, verdünnt und auf eine 4,6 × 250 mm Whatman Protesil Diphenyl-Säule gegeben. Die Proteine wurden mit einem n-Propanol-Gradienten von 20-60 % (V/V) in 0,9 M Essigsäure/0,2 M Pyridin, pH 4,0, innerhalb von 6,5 Stunden eluiert. Es wurde ein symmetrischer Peak erhalten, der mit der IL-2-Aktivität übereinstimmte. Die Ausbeute an Aktivität war auf dieser Stufe grösser als 60 %. Die Homogenität dieses Materials wurde durch analytische SDS-PAGE und durch zweidimensionale Gelelektrophorese bestätigt, die ein einzelnes Band bzw. einen einzelnen Fleck lieferten. Das so erhaltene homogene HIL-2 besass eine spezifische Aktivität von $1,4 \times 10^9$ E/mg und ein pI von 5,68.

Proben dieses Materials wurden der Aminosäureanalyse mit Fluorescamin-Nachweis nach der Methode von Stein et al. (Arch. Biochem. Biophys. *155*, 203 [1973]) unterworfen. Das Polypeptid wurde dabei 24 und 48 Stunden bei 104 °C in konstant-siedender HCl mit einem Gehalt von 0,1 % Thioglycolsäure hydrolysiert. Die Ergebnisse sind in der folgenden Tabelle 2 zusammengefasst :

(Siehe Tabelle II Seite 7 f.)

Tabelle II

|        | 24 Stunden | 24 Stunden | 48 Stunden | Aminosäure-zusammensetzung von HIL-2 |
|--------|------------|------------|------------|--------------------------------------|
| Asp    | 12.3       | 11.8       | 11.8       | 12      |
| Thr    | 8.6        | 9.6        | 7.4        | 10      |
| Ser    | 7.5        | 5.2        | 7.3        | 8       |
| Glu    | 18.3       | 15.4       | 16.0       | 16-17   |
| Pro    | 9.7        | 10.7       | -          | 8-9     |
| Gly    | 7.5        | 6.3        | 10.8       | 8       |
| Ala    | 7.5        | 6.0        | 8.4        | 7       |
| Cys    |            |            |            | 4-5*    |
| Val    | 5.0        | 5.5        | 5.2        | 6       |
| Met    | 4.3        | 3.3        | 3.1        | 4       |
| Ile    | 5.0        | 7.5        | 6.3        | 7       |
| Leu    | 13.7       | 19.4       | 16.0       | 16      |
| Tyr    | 2.4        | 1.7        | 4.2        | 3       |
| Phe    | 4.6        | 4.1        | 6.4        | 5       |
| His    | 4.4        | 3.8        | 4.9        | 4       |
| Lys    | 7.9        | 8.6        | 7.9        | 8       |
| Arg    | 3.9        | 3.9        | 7.5        | 4       |

Total 130-133

* 24 Std. Hydrolyse einer separaten Probe

Sequenzanalyse ergab bisher die folgende interne Partialaminosäuresequenz :
Ile-Leu-Asn-Gly-Ile-Asn-Asn-Tyr-Lys-Asn-Pro-Lys-Leu-Thr.
Diese Sequenz stimmt mit den Positionen 44-57 der von Taniguchi et al. (Nature *302*, 305-310 [1983]) aus klonierter cDNA abgeleiteten Aminosäuresequenz von Human-IL-2-überein.

**Patentansprüche** (für die Vertragsstaaten : BE, CH, DE, FR, GB, IT, LI, LU, NL, SE)

1. Homogenes Human-Interleukin 2 (HIL-2), gekennzeichnet durch eine spezifische Aktivität von etwa $1,4 \times 10^9$ E/mg, ein pI von 5,68 und die folgende Aminosäurezusammensetzung :

| Asp | 12   | Ala | 7   | Tyr | 3 |
|-----|------|-----|-----|-----|---|
| Thr | 10   | Cys | 4-5 | Phe | 5 |
| Ser | 8    | Val | 6   | His | 4 |
| Glu | 16-17| Met | 4   | Lys | 8 |
| Pro | 8-9  | Ile | 7   | Arg | 4 |
| Gly | 8    | Leu | 16  |     |   |

2. Homogenes Human-Interleukin 2 gemäss Anspruch 1 zur Verwendung als Arzneimittel.

3. Verfahren zur Herstellung von homogenem Human-Interleukin 2 (HIL-2), dadurch gekennzeichnet, dass man

a) eine rohe Lösung von Human-Interleukin 2 an einer Umkehrphasen-HPLC (High Performance Liquid Chromatography) Säule auf der Basis von Kieselgel, das kovalent gebundene Methyl- oder Octylgruppen enthält, unter HPLC-Bedingungen chromatographiert, wobei die HIL-2-Aktivität mit einem n-Propanol-Gradienten, 0-60 % (V/V) in einem Puffer eluiert und zu einer Fraktion vereinigt wird, und

b) diese Fraktion an einer weiteren Umkehrphasen-HPLC-Säule auf der Basis von Kieselgel, das kovalent gebundene Diphenylgruppen enthält, unter HPLC-Bedingungen chromatographiert, wobei die HIL-2-Aktivität mit einem n-Propanol-Gradienten, 20-60 %, (V/V) in einem Puffer eluiert und zu einer Fraktion vereinigt wird, und, gewünschtenfalls, die Verfahrensschritte (a) und/oder (b) wiederholt.

4. Verfahren nach Anspruch 3, dadurch gekennzeichnet, dass es sich bei der Säule in Verfahrensschritt (a) um eine Octylgruppen-enthaltende Kieselgelsäule handelt.

5. Verfahren gemäss Anspruch 4, dadurch gekennzeichnet, dass das Kieselgel der Octylsäule eine Porengrösse von etwa (150 Å) und das der Diphenylsäule eine Porengrösse von etwa (300 Å) aufweist.

6. Verfahren gemäss Anspruch 5, dadurch gekennzeichnet, dass der Puffer in Verfahrensstufen (a) und (b) 0,9 M Essigsäure/0,2 M Pyridin, pH 4,0, ist.

7. Verfahren gemäss einem der Ansprüche 3-6, dadurch gekennzeichnet, dass das rohe HIL-2 aus induzierten Kulturen maligner menschlicher Zellen stammt.

8. Pharmazeutische Präparate, dadurch gekennzeichnet, dass sie als aktives Prinzip homogenes HIL-2 enthalten.

**Patentansprüche** (für den Vertragsstaat AT)

1. Verfahren zur Herstellung von homogenem Human-Interleukin-2 (HIL-2), dadurch gekennzeichnet, dass man

(a) eine rohe Lösung von Human-Interleukin 2 an einer Umkehrphasen-HPLC (High Performance Liquid Chromatography)-Saüle auf der Basis von Kieselgel, das kovalent gebundene Methyl- oder Octylgruppen enthält, unter HPLC-Bedingungen chromatographiert, wobei die HIL-2-Aktivität mit einem n-Propanol-Gradienten, 0-60 %, (V/V) in einem Puffer eluiert und zu einer Fraktion vereinigt wird, und

(b) diese Fraktion an einer weiteren Umkehrphasen-HPLC-Säule auf der Basis von Kieselgel, das kovalent gebundene Diphenylgruppen enthält, unter HPLC-Bedingungen chromatographiert, wobei die HIL-2-Aktivität mit einem n-Propanol-Gradienten, 20-60 % (V/V) in einem Puffer eluiert und zu einer Fraktion vereinigt wird, und, gewünschtenfalls, die Verfahrensschritte (a) und/oder (b) wiederholt.

2. Verfahren nach Anspruch 1, dadurch gekennzeichnet, dass es sich bei der Säule in Verfahrensschritt (a) um eine Octylgruppen-enthaltende Kieselgelsäule handelt.

3. Verfahren nach einem der Ansprüche 1-2, dadurch gekennzeichnet, dass das Kieselgel der Octylsäule eine Porengrösse von etwa 15 nm (150 Å) und das der Diphenylsäule eine Porengrösse von etwa 30 nm (300 Å) aufweist.

4. Verfahren gemäss Anspruch 3, dadurch gekennzeichnet, dass der Puffer in Verfahrensstufen (a) und (b) 0,9 M Essigsäure/0,2 M Pyridin, pH 4,0, ist.

5. Verfahren gemäss einem der Ansprüche 1-4, dadurch gekennzeichnet, dass das rohe HIL-2 aus induzierten Kulturen maligner menschlicher Zellen stammt.

6. Verfahren nach einem der Ansprüche 1-5, dadurch gekennzeichnet, dass das homogene HIL-2 eine spezifische Aktivität von etwa $1,4 \times 10^9$ E/mg, ein pI von 5,68 und die folgende Aminosäurezusammensetzung besitzt :

| | | | | | | |
|-----|------|-----|-----|-----|-----|---|
| Asp | 12 | Ala | 7 | Tyr | 3 |
| Thr | 10 | Cys | 4-5 | Phe | 5 |
| Ser | 8 | Val | 6 | His | 4 |
| Glu | 16-17 | Met | 4 | Lys | 8 |
| Pro | 8-9 | Ile | 7 | Arg | 4 |
| Gly | 8 | Leu | 16 | | |

**Claims** (for the Contracting States : BE, CH, DE, FR, GB, IT, LI, LU, NL, SE)

1. Homogeneous human interleukin 2 (HIL-2), characterized by a specific activity of about $1.4 \times 10^9$ U/mg, a pI of 5.68 and the following amino acid composition :

| | | | | | | |
|-----|------|-----|-----|-----|-----|---|
| Asp | 12 | Ala | 7 | Tyr | 3 |
| Thr | 10 | Cys | 4-5 | Phe | 5 |
| Ser | 8 | Val | 6 | His | 4 |
| Glu | 16-17 | Met | 4 | Lys | 8 |
| Pro | 8-9 | Ile | 7 | Arg | 4 |
| Gly | 8 | Leu | 16 | | |

2. Homogeneous human interleukin 2 in accordance with claim 1 for use as a medicament.

3. A process for the manufacture of homogeneous human interleukin 2 (HIL-2), characterized by

(a) chromatographing a crude solution of human interleukin 2 on a reverse-phase HPLC (High Performance Liquid Chromatography) column based on silica gel, which contains covalently bonded methyl or octyl groups, under HPLC conditions, whereby the HIL-2 activity is eluted with a n-propanol gradient, 0-60 % (v/v), in a buffer and is pooled to one fraction, and

(b) chromatographing this fraction on a second reverse-phase HPLC column based on silica gel, which contains covalently bonded diphenyl groups, under HPLC conditions, whereby the HIL-2 activity is eluted with a n-propanol gradient, 20-60 % (v/v), in a buffer and is pooled to one fraction, and, if desired, repeating process step (a) and/or (b).

4. A process according to claim 3, characterized in that the column in process step (a) is a silica gel column containing octyl groups.

5. A process in accordance with claim 4, characterized in that the silica gel of the octyl column has a pore size of about 15 nm (150 Å) and that of the diphenyl column has a pore size of about 30 nm (300 Å).

6. A process in accordance with claim 5, characterized in that the buffer in process steps (a) and (b) is 0.9 M acetic acid/0.2 M pyridine, pH 4.0.

7. A process in accordance with any one of claims 3-6, characterized in that the crude HIL-2 is derived from induced cultures of malignant human cells.

8. A pharmaceutical preparation, characterized in that it contains homogeneous HIL-2 as the active constituent.

**Claims** (for the Contracting State AT)

1. A process for the manufacture of homogeneous human interleukin 2 (HIL-2), characterized by

(a) chromatographing a crude solution of human interleukin 2 on a reverse-phase HPLC (High Performance Liquid Chromatography) column based on silica gel, which contains covalently bonded methyl or octyl groups, and under HPLC conditions, whereby the HIL-2 activity is eluted with a n-propanol gradient, 0-60 % (v/v), in a buffer and is pooled to one fraction and

(b) chromatographing this fraction on a second reverse-phase HPLC column based on silica gel, which contains covalently bonded diphenyl groups, under HPLC conditions, whereby the HIL-2 activity is eluted with a n-propanol gradient, 20-60 % (v/v), in a buffer and is pooled to one fraction, and, if desired, repeating process step (a) and/or (b).

2. A process according to claim 1, characterized in that the column in process step (a) is a silica gel column containing octyl groups.

3. A process according to one of claims 1-2, characterized in that the silica gel of the octyl column has a pore size of about 15 nm (150 Å) and that of the diphenyl column has a pore size of about 30 nm (300 Å).

4. A process in accordance with claim 3, characterized in that the buffer in process steps (a) and (b) is 0.9 M acetic acid/0.2 M pyridine, pH 4.0.

5. A process in accordance with any one of claims 1-4, characterized in that the crude HIL-2 is derived from induced cultures of malignant human cells.

6. A process according to any one of claims 1-5, characterized in that the homogeneous HIL-2 has a specific activity of about $1.4 \times 10^9$ U/mg, a pI of 5.68 and the following amino acid composition :

| Asp | 12 | Ala | 7 | Tyr | 3 |
|-----|-----|-----|-----|-----|-----|
| Thr | 10 | Cys | 4-5 | Phe | 5 |
| Ser | 8 | Val | 6 | His | 4 |
| Glu | 16-17 | Met | 4 | Lys | 8 |
| Pro | 8-9 | Ile | 7 | Arg | 4 |
| Gly | 8 | Leu | 16 | | |

**Revendications** (pour les Etats contractants : BE, CH, DE, FR, GB, IT, LI, LU, NL, SE)

1. Interleukine 2 (HIL-2) humaine homogène, caractérisé par une activité spécifique d'environ $1,4 \times 10^9$ E/mg, un pI de 5,68 et la composition en acides aminés suivante :

| Asp | 12 | Ala | 7 | Tyr | 3 |
|-----|-----|-----|-----|-----|-----|
| Thr | 10 | Cys | 4-5 | Phe | 5 |
| Ser | 8 | Val | 6 | His | 4 |
| Glu | 16-17 | Met | 4 | Lys | 8 |
| Pro | 8-9 | Ile | 7 | Arg | 4 |
| Gly | 8 | Leu | 16 | | |

2. Interleukine 2 humaine homogène selon la revendication 1 aux fins d'application comme médicament.

3. Procédé de préparation d'interleukine 2 (HIL-2) humaine homogène, caractérisé en ce que

(a) on chromatographie une solution brute d'interleukine humaine 2 sur une colonne de HPLC (chromatographie liquide à haute performance) à inversion de phases à base de gel de silice, qui contient des groupes méthyle ou octyle liés de façon covalente, dans des conditions de HPLC, l'activité d'HIL-2 étant éluée avec un gradient de n-propanol, 0,60 % (v/v), dans un tampon et rassemblée en une fraction, et

(b) on chromatographie cette fraction sur une autre colonne de HPLC à inversion de phases à base de gel de silice, qui contient des groupes diphényle liés de façon covalente, dans des conditions de HPLC, l'activité d'HIL-2 étant éluée avec un gradient de n-propanol, 20-60 % (v/v), dans un tampon et rassemblée en une fraction, et, si on le désire, les étapes (a) et/ou (b) du procédé étant répétées.

4. Procédé selon la revendication 3, caractérisé en ce qu'il s'agit quant à la colonne de l'étape (a) du procédé d'une colonne de gel de silice contenant des groupes octyle.

5. Procédé selon la revendication 4, caractérisé en ce que le gel de silice de la colonne d'octyle présente une taille de pores d'environ 15 nm (150 Å) et en ce que celui de la colonne de diphényle présente une taille de pores d'environ 30 nm (300 Å).

6. Procédé selon la revendication 5, caractérisé en ce que le tampon dans les étapes (a) et (b) du procédé est l'acide acétique 0,9 M/pyridine 0,2 M, pH 4,0.

7. Procédé selon l'une des revendications 3-6, caractérisé en ce que l'HIL-2 brute provient de cultures induites de cellules humaines malignes.

8. Préparations pharmaceutiques, caractérisées en ce qu'elles contiennent comme principe actif l'HIL-2 homogène.

**Revendications** (pour l'Etat contractant AT)

1. Procédé de préparation d'interleukine-2 humaine homogène (HIL-2), caractérisé en ce que

(a) on·chromatographie une solution brute d'interleukine 2 humaine sur une colonne de HPLC (chromatographie liquide à haute performance) à inversion de phases à base de gel de silice, qui contient des groupes méthyle ou octyle liés de façon covalente, dans des conditions de HPLC, l'activité d'HIL-2 étant éluée avec un gradient de n-propanol, 0,60 % (v/v), dans un tampon et rassemblée en une fraction, et

(b) on chromatographie cette fraction sur une autre colonne de HPLC à inversion de phases à base de gel de silice, qui contient des groupes diphényle liés de façon covalente, dans des conditions de HPLC, l'activité de HIL-2 étant éluée avec un gradient de n-propanol, 20-60 % (v/v), dans un tampon et rassemblée en une fraction et, si on le désire, les étapes (a) et/ou (b) du procédé étant répétées.

2. Procédé selon la revendication 1, caractérisé en ce qu'il s'agit quant à la colonne dans l'étape (a) du procédé d'une colonne de gel de silice contenant des groupes octyle.

3. Procédé selon l'une des revendications 1-2, caractérisé en ce que le gel de silice de la colonne d'octyle présente une taille de pores d'environ 15 nm (150 Å) et celui de la colonne de diphényle présente une taille de pores d'environ 30 nm (300 Å).

4. Procédé selon la revendication 3, caractérisé en ce que le tampon dans les étapes (a) et (b) du procédé est l'acide acétique 0,9 M/pyridine 0,2 M, pH 4,0.

5. Procédé selon l'une des revendications 1-4, caractérisé en ce que l'HIL-2 brute provient de cultures induites de cellules humaines malignes.

6. Procédé selon l'une des revendications 1-5, caractérisé en ce que l'HIL-2 homogène présente une activité spécifique d'environ $1,4 \times 10^9$ E/mg, un pI de 5,68 et la composition en acides aminés suivante :

| | | | | | |
|-----|------|-----|-----|-----|---|
| Asp | 12   | Ala | 7   | Tyr | 3 |
| Thr | 10   | Cys | 4-5 | Phe | 5 |
| Ser | 8    | Val | 6   | His | 4 |
| Glu | 16-17| Met | 4   | Lys | 8 |
| Pro | 8-9  | Ile | 7   | Arg | 4 |
| Gly | 8    | Leu | 16  |     |   |